Europäisches Patentamt

European Patent Office

Office européen des brevets

(11) Veröffentlichungsnummer : **0 185 010**
**B1**

(12) **EUROPÄISCHE PATENTSCHRIFT**

(45) Veröffentlichungstag der Patentschrift :
26.04.89

(51) Int. Cl.⁴ : **C 12 F 3/10, C 12 P 7/06**

(21) Anmeldenummer : 85890302.4

(22) Anmeldetag : 05.12.85

(54) **Verfahren zur Kontinuierlichen Gewinnung von Fermentationsprodukten.**

(30) Priorität : 13.12.84 AT 3952/84

(43) Veröffentlichungstag der Anmeldung :
18.06.86 Patentblatt 86/25

(45) Bekanntmachung des Hinweises auf die Patenterteilung : 26.04.89 Patentblatt 89/17

(84) Benannte Vertragsstaaten :
BE CH DE FR GB IT LI LU NL SE

(56) Entgegenhaltungen :
EP—A— 0 072 045
WO—A—83 /016 27
DE—A— 3 007 138
DE—A— 3 204 910
GB—A— 1 109 311

(73) Patentinhaber : VOGELBUSCH GESELLSCHAFT m.b.H.
Blechturmgasse 11
A-1050 Wien (AT)

(72) Erfinder : Krenn, Wolfgang, Dipl.-Ing. Dr.
Jenneweingasse 32/3
A-1210 Wien (AT)

(74) Vertreter : Wolfram, Gustav, Dipl.-Ing.
Schwindgasse 7 P.O. Box 205
A-1041 Wien (AT)

EP 0 185 010 B1

**Beschreibung**

Die Erfindung betrifft ein Verfahren zur kontinuierlichen Gewinnung von Fermentationsprodukten, insbesondere Äthanol, aus fermentierter Maische, wobei die Maische aus einer gegebenenfalls auch eine Rohstoffaufbereitung umfassenden Fermentationsstufe — gegebenenfalls nach Abscheidung und Rückführung einer Mikroorganismenkultur in die Fermentationsstufe — einer Destillationseinheit zugeführt, vom Bodenteil der Destillationseinheit weitgehend von Fermentationsprodukten befreite Schlempe abgezogen und teilweise in die Rohstoffaufbereitungs- und Fermentationsstufe rückgeführt wird.

In der US-A-2 440 925 ist bereits ein Verfahren zur Alkoholerzeugung beschrieben, bei dem in einem Gärbehälter befindliche Maische mit Hefe vergoren wird, ein Teil der fermentierten Maischflüssigkeit kontinuierlich abgezogen, unter vermindertem Druck destilliert wird und die vom Alkohol befreite Schlempe wieder dem Gärbehälter zugeführt wird, um den Alkoholgehalt der Maische niedrig zu halten und somit die Hefe nicht zu schädigen. Der Produktionsprozeß muß jedoch von Zeit zu Zeit unterbrochen werden, um die mit unverwertbaren Stoffen angereicherte Maische aus dem Gärbehälter abzulassen.

In gärungstechnischen Betrieben stellt die Entsorgung der Schlempe in zunehmendem Maße ein Schwerpunktproblem dar. Eine der gängigsten Methoden ist derzeit die Konzentrierung der Schlempe und deren Verwendung als Futtermittel oder als Brennstoff — beispielsweise zur Dampferzeugung.

Um die anfallenden Schlempemengen zu reduzieren und die zur Verdünnung der Maische benötigte Menge an Prozeßwasser zu verringern, ist es bekannt, alkoholische Maischflüssigkeit mit unterschiedlichen Gehalten an Äthanol oder aber Schlempe von der Destillation in die Rohstoffaufbereitung und/oder in die Fermentation rückzuführen. Eine Rohstoffaufbereitung kann beispielsweise in einem Aufschluß und der Verzuckerung von Stärke bestehen. Auch eine allfällige Abtrennung und Ausschleusung von suspendierten Feststoffen mit Trennvorrichtungen wie Bogensieben, Absetzbehältern und Zentrifugen sowie die Separation von Hefe aus alkoholischer Maische und Rückführung der Hefe in die Fermentationsstufe sind bekannt.

So betrifft die EP-B1-0 011 334 ein Verfahren zur kontinuierlichen Herstellung von Äthanol, wobei aus der Fermentierung ein Teilstrom der vergorenen Maische kontinuierlich abgezogen wird, die Hefezellen aus der Maische mittels einer Zentrifuge abgetrennt und wieder in die Fermentation eingebracht werden und die Maische nach vollständigem oder teilweisem Entzug des Alkohols in einem Vakuumverdampfer in die Fermentation rückgeführt wird.

Nach der DE-A-29 46 161 wird eine Strömung aus der Fermentierung durch Zentrifugieren in ein Hefekonzentrat und eine hefefreie Strömung geteilt, das Hefekonzentrat wieder der Fermentierung zugeführt und die hefefreie Strömung destilliert. Die bei der Destillation resultierende äthanolarme Restströmung wird teilweise, nachdem sie zur Aufbereitung einer Rohmaterialströmung eingesetzt wurde, als von Feststoffen befreite Substratströmung wieder in die Fermentierung rückgeführt.

Auch gemäß der DE-A-30 07 138 wird ein Hefekonzentrat von der Gärflüssigkeit abgetrennt und in die Fermentation rückgeführt ; die hefefreie äthanolhältige Flüssigkeit wird zunächst in einen einfachen Verdampfer gespeist und dort in eine äthanolreiche Dampfströmung und eine Bodenströmung aufgeteilt. Der größere Teil der flüssigen Bodenströmung wird in die Fermentation rückgeführt und der Restteil einer Abtriebeinheit zugeführt, von der das äthanolreiche Kopfprodukt gemeinsam mit der äthanolreichen Dampfströmung vom Verdampfer in eine Destilliereinheit gelangt.

Nach der DE-A-32 04 910 wird das anfallende Schlempewasser teilweise in die Rohstoffaufbereitung zur Bildung von Süßmaische rückgeführt. Dabei wird ein Teil des Schlempewassers in einem Rückflußerzeuger sowie in einem daran angeschlossenen Verdichter in hochgespannten Dampf mit einem Druck von etwa 10 bar umgewandelt, mit welchem Süßmaische erwärmt wird. Ein nicht zur Erwärmung benötigter Rest an hochgespanntem Dampf aus dem Verdichter wird zur Beheizung der Destillationsanlage verwendet.

Nach der WO-A-83 01 627 wird die Maische in einem Verdampfer und in einer Destillationskolonne aufgearbeitet. Die Abtrennung des Äthanols gelingt allerdings nicht vollständig, so daß der rückgeführte Schlempestrom noch äthanolhältig ist.

Nach der GB-A-1 109 311 geschieht die Aufarbeitung der Maische in drei Stufen : In einer ersten Destillationskolonne wird äthanolhältiger Dampf abgetrennt und die entstehende Schlempe in zwei nachgeschalteten Verdampfern voreingedampft und aufkonzentriert, wobei die Kolonne indirekt beheizt wird.

Aus der DE-A-30 23 120 ist es zur Herstellung von Äthanol durch Fermentieren eines kohlenhydrathältigen Substrates bekannt, die Fermentierungsbrühe vorerst einem einfachen Vakuumverdampfer zuzuführen, die flüssige, hefehaltige Bodenströmung aus dem Verdampfer zum Teil in die Fermentierung zurückzuführen und den Rest in eine Hefekonzentratströmung, welche gleichfalls in die Fermentierung zurückgeführt wird, sowie in eine hefefreie Strömung, welche einer Abstreifeinheit zugeführt wird, aufzuteilen. Die äthanolreiche Dampfströmung aus dem Vakuumverdampfer wird in einer Rektifizierkolonne weiter aufgearbeitet, die äthanolreiche Dampfströmung aus der Abstreifeinheit kann dem Verdampfer zugeführt werden.

Die DE-A-30 36 872 schließlich beschäftigt sich mit einem Verfahren ähnlicher Art mit integrierter

EP 0 185 010 B1

Rohstoffaufbereitung, bei dem die Gärbrühe in eine rückgeführte Hefekonzentratströmung und eine hefefreie Strömung aufgeteilt wird, zumindest ein Teil der hefefreien Strömung in einem einfachen Verdampfer in eine äthanolreiche Dampfströmung und eine flüssige Bodenströmung aufgeteilt, letztere wenigstens teilweise erneut der Fermentation zugeführt und die äthanolreiche Dampfströmung in eine Fraktioniereinheit geleitet wird. Der polysaccharidhaltige Rohstoff wird — vorzugsweise vorhydrolysiert — in den Verdampfer eingebracht und dort enzymatisch weiter hydrolysiert. Mit der flüssigen Bodenströmung gelangen die aufgeschlossenen Rohstoffe in die Fermentation, wobei auch dem Fermentor Enzyme zugegeben werden können.

Wird nach den bekannten Verfahren lediglich teilweise entalkoholisierte Maische rückgeführt, ergibt sich aufgrund der kreislaufgeführten Alkoholmenge eine entsprechende Vergrößerung der Flüssigkeitsvolumina in der Fermentation und in eventuellen Separationsvorrichtungen. Größer auszulegende Apparate bedingen jedoch erhöhte Investitionskosten und verbrauchen beim Betrieb mehr Energie.

Wird hingegen weitgehend entalkoholisierte Maische (Schlempe) rückgeführt, welche den gleichen Gehalt an Trockensubstanzen aufweist wie die aus dem Prozeß abzuleitende Schlempe, fallen immer noch verhältnismäßig große Schlempemengen an, da die rückführbare Schlempemenge bei den meisten Substraten durch eine maximale Trockensubstanzkonzentration in der Fermentationsstufe begrenzt wird. Abgesehen davon ist es prozeßtechnisch in jedem Fall günstiger, in der zu fermentierenden Maische einen möglichst geringen Anteil an unvergärbarer Trockensubstanz vorzusehen.

Die Erfindung bezweckt, die geschilderten, mit den bekannten Verfahren noch verbundenen Nachteile und Schwierigkeiten zu überwinden und ein energiesparendes, apparativ wenig aufwendiges Verfahren zu schaffen, bei dem eine höher konzentrierte Schlempe in reduzierter Menge anfällt und die nötige Prozeßwassermenge vergleichsweise sehr gering gehalten werden kann, ohne eine erhebliche Hemmung des Fermentationsverlaufes infolge zu starker Anreicherung von Fermentationsprodukten oder unverwertbaren Substanzen in Kauf nehmen zu müssen.

Die gestellte Aufgabe wird bei einem Verfahren der eingangs definierten Art erfindungsgemäß dadurch gelöst, daß ein weiterer Teilstrom der aus der Destillationseinheit abgezogenen Schlempe in einer indirekt beheizten Verdampferstufe aufkonzentriert wird, die in der Verdampferstufe entstehenden Brüden direkt in den unteren Bereich der Destillationseinheit eingeleitet werden und die aufkonzentrierte Schlempe aus der Verdampferstufe abgeleitet wird.

Neben Äthanol können auch andere destillierbare Fermentationsprodukte, wie Butanol und Aceton, als Kopfprodukte einer Destillationseinheit gewonnen werden. Butanol und gegebenenfalls Aceton entstehen bei der anaeroben Fermentation mit beispielsweise Clostridium butylicum oder acetobutylicum aus glucosehältigen Substraten. Zur äthanolischen Gärung werden als Mikroorganismen üblicherweise Hefen eingesetzt.

Die Abscheidung der Mikroorganismenkultur aus der fermentierten Maische kann bereits vor Einspeisung der fermentationsprodukthältigen Maische in die Destillationseinheit — beispielsweise mittels Zentrifugieren — erfolgen. Wird in der Destillationseinheit nicht unter ausreichend reduziertem Druck gearbeitet, so daß bei der Destillation Temperaturen über etwa 40 °C erreicht werden, die für die Mikroorganismen bereits schädlich sind, ist deren vorangehende Abscheidung natürlich in vielen Fällen vorzuziehen.

Nach einer vorteilhaften Ausführungsform des erfindungsgemäßen Verfahrens werden in der abgezogenen Schlempe suspendierte Stoffe abgetrennt. Erst die von suspendierten Feststoffen befreite Schlempe wird aufgeteilt, teilweise in die Rohstoffaufgbereitung und/oder in die Fermentationsstufe rückgeführt und ein anderer Teilstrom wird in die Verdampferstufe geleitet.

Für den Fall einer unter ausreichendem Vakuum betriebenen Destillationseinheit kann die Mikroorganismenkultur auch erst aus der abgezogenen Schlempe — beispielsweise mittels Zentrifugalseparatoren — abgetrennt und wieder der Fermentationsstufe zugesetzt werden.

Betreibt man die Destillationseinheit unter nur geringem Vakuum, unter Normal- oder Überdruck, wird zweckmäßig der Wärmeinhalt der aus der Destillationseinheit abgezogenen Schlempe in einem oder mehreren Wärmeaustauschern teilweise auf die der Destillationseinheit zugeführte Maische übertragen. Die Wärmeübertragung auf die von der Fermentationsstufe kommende Maische wird in diesem Fall inbesondere auch vor der Abtrennung suspendierter Stoffe aus der Schlempe, also unmittelbar nach dem Abziehen der Schlempe aus der Destillationseinheit (Trennkolonne) vorgenommen, um mit dem Siedeverhalten der Schlempe zusammenhängende Störungen — beispielsweise bei einer Abtrennung durch Dekantation — sowie Wärmeverluste bei einer eventuellen Zwischenlagerung zu vermeiden. Zwecks noch besserer Energieausnützung ist es zusätzlich von Vorteil, auch den von suspendierten Stoffen befreiten, in die Verdampferstufe zu leitenden Teilstrom der Schlempe mittels der Gesamtmenge der unmittelbar aus der Destillationseinheit abgezogenen Schlempe vorzuwärmen, wobei ein Teil des Wärmeinhaltes der noch suspendierte Stoffe enthaltenden Schlempe zunächst auf diesen Teilstrom und danach auf die der Destillationseinheit zugeführten Maische übertragen wird.

Wird in der Destillationseinheit ein ausreichend niedriger Druck eingestellt, so ist eine Abkühlung der Gesamtmenge der abgezogenen Schlempe nicht erforderlich und es wird vorzugsweise nur der in die Rohstoffaufbereitung und/oder in die Fermentationsstufe rückgeführte Teil der Schlempe zur Maischevorwärmung verwendet. Die Vorwärmung der Maische kann zusätzlich oder ausschließlich auch mit irgendwelchen anderen, im Betrieb anfallenden, warmen Medien vorgenommen werden.

3

Eine bevorzugte Ausführungsform der Erfindung wird im folgenden anhand eines Verfahrensschemas näher erläutert.

Ein wässeriger Rohstoffstrom 1 mit pflanzlichem, Zucker-, Stärke- und Cellulose-hältigem Ausgangsmaterial, wie Getreide, Zuckerrohr oder Melasse gelangt zunächst in eine Aufbereitung 2, in welcher die Rohstoffe aufgeschlossen, hydrolysiert und notwendigenfalls gereinigt werden. Das erhaltene Substrat bzw. die Maische 3 wird in die Fermentationsstufe 4 geleitet, wo unter Einwirkung von Mikroorganismen die Fermentierung oder Gärung stattfindet.

Eine bestimmte Fraktion der fermentierten Maische wird kontinuierlich abgezogen und durch die Leitung 5 in einen Zentrifugalseparator 6 geleitet. Die abgeschiedene Mikroorganismenkultur wird durch die Leitung 7 in die Fermentationsstufe 4 rückgeführt und die fermentationsprodukthältige Maische wird durch einen Regenerativwärmeaustauscher 8 in den oberen Abschnitt einer Destillationseinheit 9 gepumpt. Die Destillationseinheit umfaßt in den meisten Fällen zweckmäßig eine einzige Trennkolonne, von deren Kopf eine mit Fermentationsprodukten angereicherte Dampfströmung durch die Leitung 10 abgezogen wird, um beispielsweise einer Rektifizierung zugeführt zu werden.

Wenn z. B. sehr große Mengen von fermentierter Maische aufgearbeitet werden müssen, kann die Destillationseinheit auch aus zwei oder mehreren Destillationskolonnen bestehen. Die weitgehend von Fermentationsprodukten befreite Schlempe wird vom Bodenteil der Destillationseinheit 9 in eine Einrichtung zur Abtrennung suspendierter Stoffe geleitet. Bei der dargestellten Ausführungsform ist zur Abtrennung ein Bogensieb 11 vorgesehen.

Die abgetrennten Feststoffe werden — angedeutet durch den Pfeil 12 — aus dem Prozeß ausgeschleust. Ein Teil der mittels des Bogensiebes 11 von suspendierten Stoffen weitgehend befreiten Schlempe wird unter Wärmeabgabe an die fermentationsprodukthaltige Maische im Wärmeaustauscher 8 durch die Leitung 13 in die Rohstoffaufbereitung 2 und/oder in die Fermentationsstufe 4 rückgeführt. Ein anderer Teilstrom der Schlempe wird durch den Leitungszweig 14 in eine indirekt beheizte, allgemein mit 15 bezeichnete Verdampferstufe geführt.

Die einen oder mehrere einfache Verdampfer mit jeweils einer oder nur wenigen theoretischen Trennstufe(n) umfassende Verdampferstufe kann beispielsweise mit Frischdampf oder durch Wärmeaustausch mit irgendeinem im Betrieb zur Verfügung stehenden heißen Medium beheizt werden. Die einzustellenden Temperaturen hängen vor allem von den in den einzelnen Anlagenteilen herrschenden Druckverhältnissen ab. Die in der Verdampferstufe 15 entstehenden, im wesentlichen aus Wasserdampf bestehenden Brüden werden zur Direktbeheizung der Destillationseinheit 9 durch die Zuführung 16 in deren unteren Bereich eingeleitet.

Infolge der Brüdenabgabe ist die schließlich durch die Ableitung 17 die Verdampferstufe 15 verlassende Schlempe im Vergleich zum durch die Leitung 13 in die Rohstoffaufbereitungs- und Fermentationsstufe rückgeführten Teil der Schlempe stark aufkonzentriert. Die aufkonzentrierte Schlempe aus der Ableitung 17 kann in manchen Fällen sogar ohne weiteren Wasserentzug als Futtermittel verwertet oder, da sie aufgrund ihres hohen Trockensubstanzgehaltes autothermisch ist, direkt verbrannt werden.

Beim erfindungsgemäßen Verfahren werden somit auf besonders energiesparende Weise nicht fermentierbare Stoffe sowie Fermentationsprodukte, welche in höheren Konzentrationen für die Mikroorganismen toxisch sind, laufend aus dem Prozeß entfernt und darüber hinaus wird die anfallende Schlempemenge drastisch reduziert.

In der folgenden Tabelle werden am Beispiel der äthanolischen Gärung durch Gegenüberstellung von Stoffströmen in zwei bekannten Verfahren und beim erfindungsgemäßen Verfahren die Vorteile des erfindungsgemäßen Verfahrens demonstriert.

I bezieht sich auf ein bekanntes Verfahren, bei dem die Destillationseinheit im Bodenteil indirekt beheizt wird, teilweise entalkoholisierte Maische in die Rohstoffaufbereitungs- und Fermentationsstufe rückgeführt wird und Schlempe vom Sumpf der Destillationseinheit abgezogen wird.

II symbolisiert ein bekanntes Verfahren, bei dem die Beheizung der Destillationseinheit wie bei Verfahren I erfolgt, jedoch weitgehend entalkoholisierte Maische (Schlempe) vom Sumpf der Destillationseinheit teilweise rückgeführt wird.

III bezeichnet das erfindungsgemäße Verfahren.

Die Zusammensetzung der von der Fermentationsstufe zugeleiteten fermentierten Maische war in allen drei Fällen gleich. Als Bezugsbasis wurde die Gewinnung von 100 kg reinem Äthanol/h gewählt, wobei der Äthanolgehalt des vom Kopf der Destillationseinheit abgezogenen Rohsprits jeweils 40 Massen% beträgt.

Wie aus der Tabelle hervorgeht, ist beim erfindungsgemäßen Verfahren (III) die zu verarbeitende Gesamtmenge an fermentierter Maische um etwa 11 % geringer als nach dem Verfahren I. Die Gesamtmenge an Schlempe ist beim erfindungsgemäßen Verfahren — verglichen mit Verfahren II — um etwa 30 % reduziert bei gleichzeitig höherem Gehalt an Trockensubstanz.

(Siehe Tabelle Seite 5 f.)

4

Tabelle

| Stoffstrom | fermentierte Maische | | | Rohsprit vom Kopf der Destillationseinheit | | | Rückführung | | | Schlempe | | |
|---|---|---|---|---|---|---|---|---|---|---|---|---|
| | I | II | III | I | II | III | I | II | III | I | II | III |
| Äthanol Massen% | 6 | 6 | 6 | 40 | 40 | 40 | 1 | ±0 | ±0 | ±0 | ±0 | ±0 |
| kg/h | 112,5 | 100 | 100 | 100 | 100 | 100 | 12,5 | ±0 | ±0 | ±0 | ±0 | ±0 |
| Trockensubstanz Massen% | 16 | 16 | 16 | 0 | 0 | 0 | 16 | 18,9 | 16 | 26,7 | 18,9 | 26,7 |
| kg/h | 300 | 266,7 | 266,7 | 0 | 0 | 0 | 200 | 166,7 | 166,7 | 100 | 100 | 100 |
| Gesamtmenge kg/h | 1875 | 1666,7 | 1666,7 | 250 | 250 | 250 | 1250 | 885,5 | 1041,7 | 375 | 529,1 | 375 |

EP 0 185 010 B1

# EP 0 185 010 B1

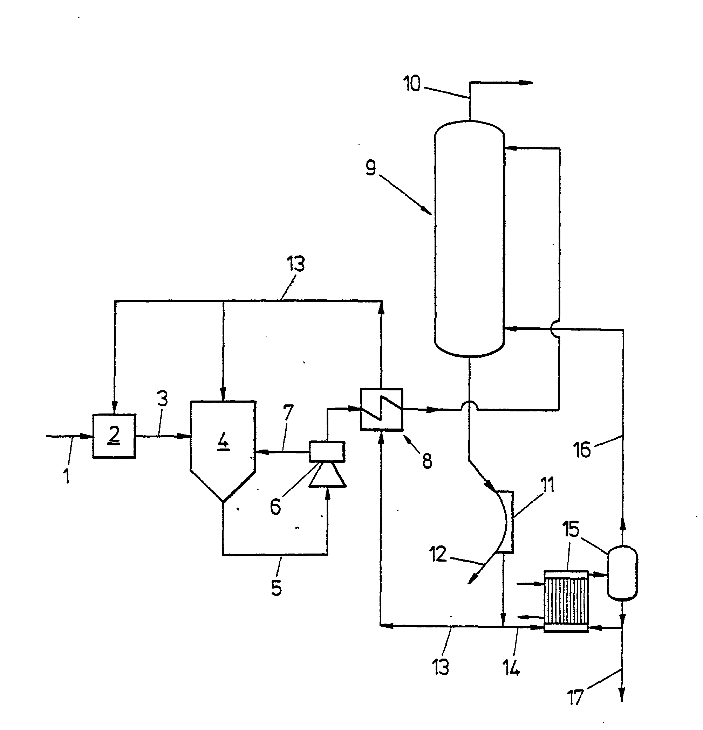

## Patentansprüche

1. Verfahren zur kontinuierlichen Gewinnung von Fermentationsprodukten, insbesondere Äthanol, aus fermentierter Maische, wobei die Maische aus einer gegebenenfalls auch eine Rohstoffaufbereitung umfassenden Fermentationsstufe — gegebenenfalls nach Abscheidung und Rückführung einer Mikroorganismenkultur in die Fermentationsstufe — einer Destillationseinheit zugeführt, vom Bodenteil der Destillationseinheit weitgehend von Fermentationsprodukten befreite Schlempe abgezogen und teilweise in die Rohstoffaufbereitung und/oder in die Fermentationsstufe rückgeführt wird, dadurch gekennzeichnet, daß ein weiterer Teilstrom der aus der Destillationseinheit abgezogenen Schlempe in einer indirekt beheizten Verdampferstufe aufkonzentriert wird, die in der Verdampferstufe entstehenden Brüden direkt in den unteren Bereich der Destillationseinheit eingeleitet werden und die aufkonzentrierte Schlempe aus der Verdampferstufe abgeleitet wird.

2. Verfahren nach Anspruch 1, dadurch gekennzeichnet, daß in der abgezogenen Schlempe suspendierte Stoffe abgetrennt werden.

3. Verfahren nach den Ansprüchen 1 und 2, dadurch gekennzeichnet, daß der Wärmeinhalt der aus der Destillationseinheit abgezogenen Schlempe in einem oder mehreren Wärmeaustauschern teilweise auf die der Destillationseinheit zugeführte Maische übertragen wird.

## Claims

1. Method of continuously recovering fermentation products, in particular ethanol, from a fermented mash, including supplying the mash from a fermentation plant, possibly also comprising a raw material processing station — possibly after separation and return of a microorganism culture into the fermentation plant — to a distilling unit, drawing off distillery slops largely freed from fermentation products from the bottom part of the distilling unit and partially returning the distillery slops into the raw material processing station and/or the fermentation plant, characterised in that a further partial flow of the distillery slops drawn off from the distilling unit is concentrated in an indirectly heated evaporator stage, the vapours formed in the evaporator stage are directly introduced into the lower region of the distilling unit and the concentrated distillery slops are discharged from the evaporator stage.

2. Method according to claim 1, characterised in that substances suspended in the drawn-off distillery slops are separated.

3. Method according to claims 1 and 2, characterised in that, in one or more heat exchangers, the thermal content of the distillery slops drawn off from the distilling unit is partially transferred onto the mash supplied to the distilling unit.

## Revendications

1. Procédé pour récupérer en continue des produits de fermentation, en particulier l'éthanol, de moûts fermentés, dans lequel le moût provenant d'un stade fermentation comprenant le cas échéant également une préparation de la matière première — éventuellement après séparation et recyclage d'une culture de micro-organismes au stade fermentation — est envoyé à une unité de distillation d'où l'on évacue, au fond, des vinasses pratiquement débarrassées des produits de fermentation qu'on recycle en partie à la préparation de la matière première et/ou au stade fermentation, ce procédé se caractérisant en ce qu'un autre courant partiel des vinasses évacuées de l'unité de distillation est concentré dans un stade vaporisation chauffé par chauffage indirect, les vapeurs formées au stade vaporisation sont envoyées directement dans la région inférieure de l'unité de distillation, et les vinasses concentrées sont évacuées du stade vaporisation.

2. Procédé selon la revendication 1, caractérisé en ce que l'on sépare les matières en suspension dans les vinasses évacuées.

3. Procédé selon les revendications 1 et 2, caractérisé en ce que la chaleur contenue dans les vinasses évacuées de l'unité de distillation est transférée en partie, dans un ou plusieurs échangeurs de chaleur, au moût envoyé à l'unité de distillation.